# EUROPEAN PATENT APPLICATION

(11) **EP 3 361 463 A1**
(43) Date of publication of application: **15.08.2018**
(21) Application number: 16823609.9
(22) Date of filing: 18.03.2016
(51) Int. Cl.: G08B 21/20, A61F 13/42

(54) **DIAPER ALARM DEVICE**

(30) Priority: 16.07.2015 CN 201520513917 U
(71) Applicant: Shenzhen Hyan Microelectronics Co., Ltd., Shenzhen Guangdong 518108 (CN)
(72) Inventor: TENG, Yujie, Shenzhen Guangdong 518108 (CN); TENG, Yudong, Shenzhen Guangdong 518108 (CN)
(74) Representative: Hellmich, Wolfgang
(86) International application number: PCT/CN2016/000144
(87) International publication number: WO 2017/008458

(57) **Abstract**

A diaper alarm device, comprises a shell, a circuit board mounted in the shell, and a connection mechanism configured to detachably connect a diaper sensor, wherein the shell comprises a plate body with a receiving cavity, a main cover with a downward concave arc surface on one end, and a slave cover with a cam surface on one end. A mouth portion of the receiving cavity is arranged on one plate surface of the plate body. The main cover and the slave cover are arranged on the mouth portion of the receiving cavity side by side. In addition, the cam surface is in sliding contact with the concave arc surface. A chute in which a metal shaft is mounted is formed in the bottom of the slave cover. Two ends of the metal shaft, which are bent, are then pin-connected to the plate body or the circuit board, such that the slave cover is limited in the mouth portion of the receiving cavity and cannot rotate until suffering an external force. The connection mechanism comprises connection electrodes arranged on the circuit board, and a plurality of short pins arranged on the lower surface of the slave cover, wherein the connection electrodes are located below the slave cover and correspond to the plurality of short pins, such that the diaper sensor and the diaper alarm device can be detachably connected conveniently, rapidly and reliably.

## Description

### FIELD OF THE INVENTION

The present invention relates to an intelligent diaper, and in particular, to a diaper alarm device of the intelligent diaper.

### BACKGROUND OF THE INVENTION

The diapers of the present invention are broadly referred to as various paper diapers and paper urine trousers. Paper diapers and paper urine trousers have been used more and more widely because of their ease of use, and have become common items for babies and the elderly. However, it is impossible for the traditional diaper to judge from the appearance whether a wearer has defecated or not, and a caregiver needs to observe it frequently, thereby often causing the situation that the wearer is uncomfortable because the diaper is not changed in time. By providing a sensor in the diaper, a warning signal can be sent after the wearer has defecated to make a prompt for the caregiver, and therefore the above problems can be overcome.

Because of high cost of a circuit portion (i.e., a diaper alarm device or a communication trigger), the diaper is often a disposable item. If the sensor and the circuit portion are integrated, there will be the following defects: 1, the use cost is very high if the sensor and the circuit portion are discarded after once use together with the diaper; 2, the sensor portion and the circuit portion are difficult to clean if they are detached from the diaper, cleaned and then reused. In order to solve the above-mentioned problems, the sensor and the circuit portion designed separately, i.e., the sensor and the circuit portion may be adhered to the surface of the diaper respectively, and then are electrically connected together in a detachable connection manner, such that the sensor and the diaper which are low in price serve as disposable items, but the circuit portion which is relatively high in cost may be reused.

However, because the diaper sensor is usually made of cloth or paper, is relatively soft, very small in thickness (generally, 1 mm or less) and easily bent, and is strip-shapedintegrally, the defects of difficulty and unreliability in connection will be caused if a common detachable connection manner (e.g., plugging) is adopted.

### SUMMARY OF THE INVENTION

The present invention aims to provide a diaper alarm device so as to solve the problems of difficulty and unreliability in connection when the diaper sensor and the diaper alarm device are detachably connected.

The specific technical solution of the present invention is as follows.

A diaper alarm device comprises a shell, a circuit board mounted in the shell, and a connection mechanism configured to detachably connect a diaper sensor, wherein the shell comprises a plate body with a receiving cavity, a main cover with a downward concave arc surface on one end, and a slave cover with a cam surface on one end; a mouth portion of the receiving cavity is arranged on one plate surface of the plate body; the main cover and the slave cover are arranged on the mouth portion of the receiving cavity side by side; the cam surface is in sliding contact with the concave arc surface; a chute in which a metal shaft is mounted is formed in the bottom of the slave cover; two ends of the metal shaft, which are bent, are then pin-connected to the plate body or the circuit board, such that the slave cover is limited in the mouth portion of the receiving cavity and cannot rotate until suffering an external force; the connection mechanism comprises connection electrodes arranged on the circuit board, and a plurality of short pins arranged on the lower surface of the slave cover, wherein the connection electrodes are located below the slave cover and correspond to the plurality of short pins.

During use, the slave cover is overturned, such that a part of the receiving cavity is opened and the connection electrodes are exposed. One end of the diaper sensor extends into the receiving cavity, and conductive connection portions on the diaper sensor cover the connection electrodes on the circuit board in a one-to-one correspondence manner. Then, the slave cover is overturned reversely to close the receiving cavity. At this time, the short pins on the slave cover contact the conductive connection portions on the diaper sensor, such that the conductive connection portions are reliably connected to the connection electrodes on thecircuit board. In addition, under a tension of the metal shaft and under the actions of the cam surface and the concave arc surface, the slave cover is limited in the mouth portion of the receiving cavity and cannot rotate until suffering an external force, and therefore the slave cover is kept at a closed state.

Optionally, the circuit board is provided with three connection electrodes, and the slave cover is provided with three groups of short pins which are in one-to-one correspondence with the three connection electrodes.

Optionally, one end of the plate body is provided with a long hole through which the diaper sensor passes.

Optionally, the width of the receiving cavity is adaptive with the width of the diaper sensor.

Optionally, the plurality of short pins (43) has elasticity.

The present invention has the following beneficial effects: the diaper sensor and the diaper alarm device can be detachably connected conveniently, rapidly and reliably due to the adoption of the connection mechanism and the shell.

### BRIEF DESCRIPTION OF THE DRAWINGS

Fig. 1 is a schematic diagram that some embodiments of the diaper alarm device are in an exploded state;
Fig. 2 is a schematic structural diagram of a plate body which forms a shell of the diaper alarm device;
Fig. 3 is a schematic structural diagram of a main cover of the diaper alarm device;
Figs. 4-5 are schematic structural diagrams of a slave cover of the diaper alarm device, in which Fig. 4 is a schematic diagram of the end of the slave cover, and Fig. 5 is a structural diagram of the bottom of the slave cover;
Fig. 6 is a schematic diagram after the slave cover is opened;
Fig. 7 is a schematic structural diagram of a circuit board of the diaper alarm device; and
Fig. 8 is a schematic structural diagram of a plate body that forms a shell in some other embodiments.

### DETAILED DESCRIPTION OF THE PREFERRED EMBODIMENTS

The present invention will be further described below in conjunction with the drawings and embodiments.

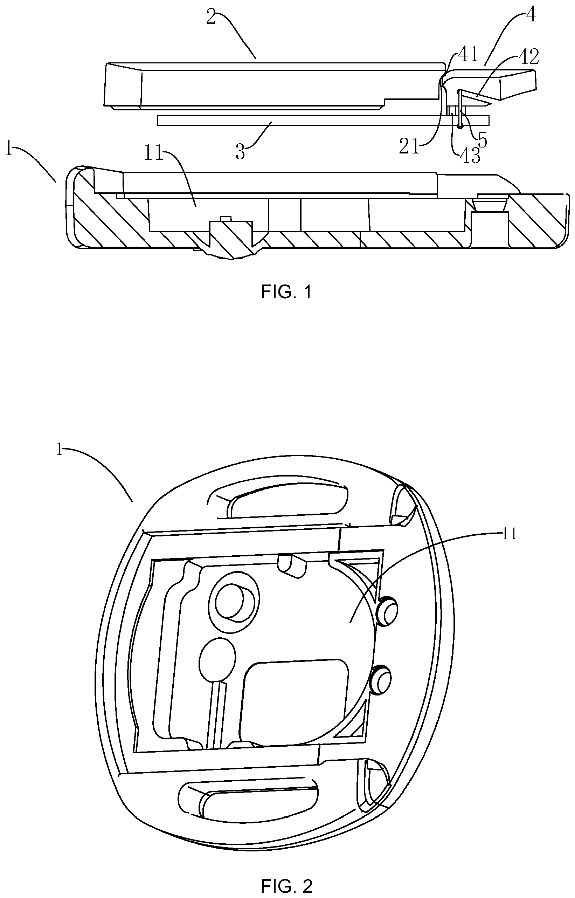

Figs. 1-7 exemplarily illustrate the structures of some embodiments of a diaper alarm device. As shown in Figs. 1-7, the diaper alarm device in some embodiments comprises a shell, a circuit board 3 mounted in the shell, and a connection mechanism configured to detachably connect a diaper sensor. The shell comprises a plate body 1 with a receiving cavity 11, a main cover 2 with a downward concave arc surface 21 on one end, and a slave cover 4 with a cam surface 41 on one end. A mouth portion of the receiving cavity 11 is arranged on one plate surface of the plate body 1. The main cover 2 and the slave cover 4 are arranged on the mouth portion of the receiving cavity side by side. The cam surface 41 is in sliding contact with the concave arc surface 21. A chute 42 in which a metal shaft 5 is mounted is formed in the bottom of the slave cover 4. The tail ends of the metal shaft 5, which are bent, are then pin-connected to the circuit board 3, such that the slave cover 4 is limited in the mouth portion of the receiving cavity 11 and cannot rotate until suffering an external force. The connection mechanism comprises connection electrodes 31 arranged on the circuit board 3, and a plurality of short pins 43 arranged on the lower surface of the slave cover 4, wherein the connection electrodes 31 are located below the slave cover 4 and correspond to the plurality of short pins 43. It may be understood that the tail end of the metal shaft 5 may also be pin-connected to the plate body 1.

During use, the slave cover 4 is overturned, such that a part of the receiving cavity 11 is opened and the connection electrodes 31 are exposed. One end of the diaper sensor extends into the receiving cavity 11, and conductive connection portions on the diaper sensor cover the connection electrodes 31 on the circuit board 3 in a one-to-one correspondence manner. Then, the slave cover 4 is overturned reversely to close the receiving cavity 11. At this time, the short pins 43 on the slave cover 4 contact the conductive connection portions on the diaper sensor, such that the conductive connection portions are reliably connected to the connection electrodes 31 on the circuit board 3. In addition, under a tension of the metal shaft 5 and under the actions of the cam surface 41 and the concave arc surface 21, the slave cover 4 is limitedin the mouth portion of the receiving cavity 11 and cannot rotate until suffering an external force, and therefore the slave cover 4 is kept at a closed state. When the diaper alarm device needs to be separated from the diaper sensor, the diaper sensor can be removed from the diaper alarm device just by overturning the slave cover 4. It can thus be seen that the diaper sensor can be connected with or detached from the diaper alarm device conveniently, rapidly and reliably.

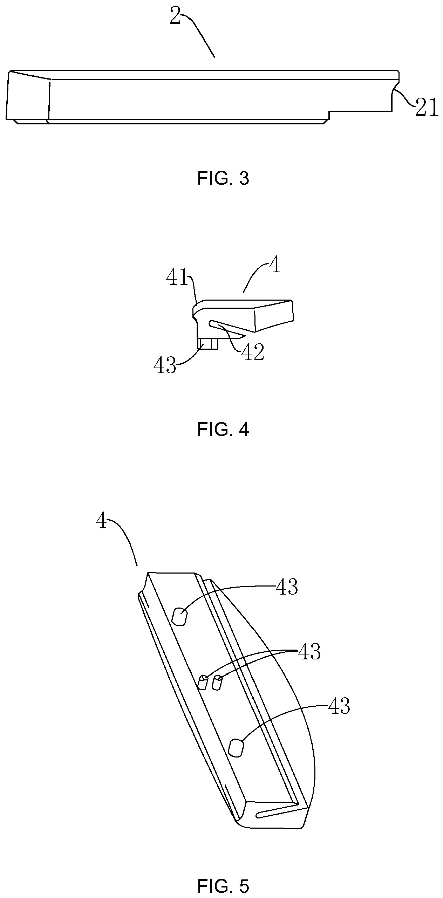

Further, the circuit board 3 is provided with three connection electrodes 31, and the slave cover 4 is provided with three groups of short pins 43. Specifically, as shown in Fig. 5, one upper short pin 43 is considered as one group, the middle two short pins 43 are considered as one group, and one bottom short pin 43 is considered as one group. The three groups of short pins 43 are in one-to-one correspondence with the three connection electrodes 31, thereby ensuring that the three connection electrodes 31 are reliably connected with the three conductive connection portions on the diaper sensor respectively. In this case, the diaper sensor is provided with three detection electrodes, which can not only detect whether a wearer has defecated or not, but also distinguish excrement from urine.

The diaper sensor can be positioned rapidly through the width of the receiving cavity 11. Specifically, the width of the receiving cavity 11 is enabled to be adaptive with the width of the diaper sensor.

Further, the plurality of short pins 43 has elasticity.

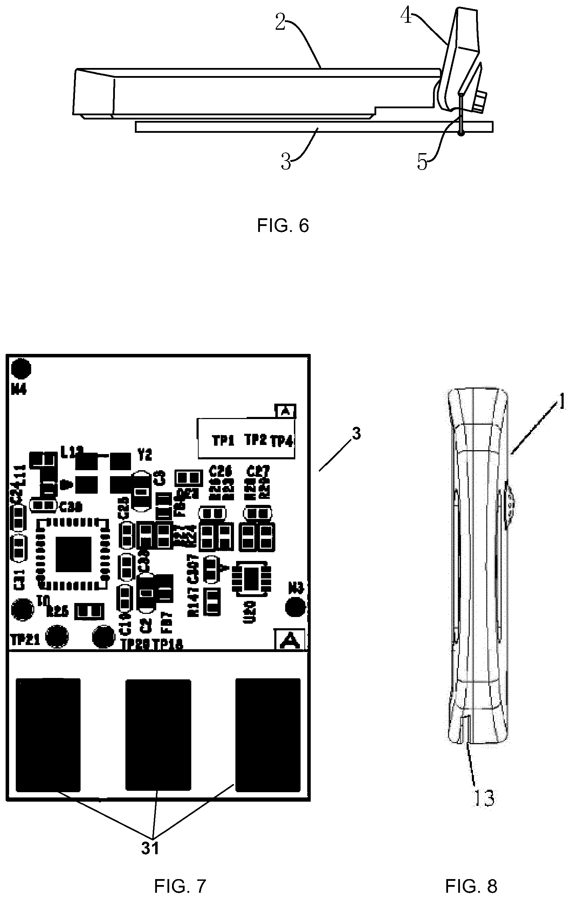

Fig. 8 illustrates a structure of a plate body 1 that forms a shell in some other embodiments. As shown in Fig. 8, one end of the plate body 1 is provided with a long hole 13 through which the diaper sensor passes. During connection, the connection end of the diaper sensor passes through the long hole 13 and then enters the receiving cavity 11, and is connected with the connection electrodes 31 on the circuit board 3. The diaper sensor can be positioned rapidly by the long hole 13.

The present invention has been described in detail with reference to specific embodiments, but these specific descriptions should not be construed as limiting the protection scope of the present invention, and those skilled in the art can also make other modifications or equivalent substitutions according to the description of the present invention. The protection scope of the present invention is subject to the claims.

## Claims

1. A diaper alarm device, comprising a shell, a circuit board mounted in the shell, and a connection mechanism configured to detachably connect a diaper sensor, wherein
the shell comprises a plate body (1) with a receiving cavity (11), a main cover (2) with a downward concave arc surface (21) on one end, and a slave cover (4) with a cam surface (41) on one end; a mouth portion of the receiving cavity is arranged on one plate surface of the plate body (1); the main cover (2) and the slave cover (4) are arranged on the mouth portion of the receiving cavity side by side; the cam surface is in sliding contact with the concave arc surface; a chute (42) in which a metal shaft (5) is mounted is formed in the bottom of the slave cover; two ends of the metal shaft, which are bent, are then pin-connected to the plate body (1) or the circuit board, such that the slave cover (4) is limited in the mouth portion of the receiving cavity and cannot rotate until suffering an external force;
the connection mechanism comprises connection electrodes (31) arranged on the circuit board (3), and a plurality of short pins (43) arranged on the lower surface of the slave cover (4), wherein the connection electrodes are located below the slave cover and correspond to the plurality of short pins.

2. The diaper alarm device according to claim 1, wherein the circuit board (3) is provided with three connection electrodes (31), and the slave cover (4) is provided with three groups of short pins (43) which are in one-to-one correspondence with the three connection electrodes.

3. The diaper alarm device according to claim 1, wherein one end of the plate body (1) is provided with a long hole (13) through which the diaper sensor passes.

4. The diaper alarm device according to claim 1, wherein the width of the receiving cavity (11) is adaptive with the width of the diaper sensor.

5. The diaper alarm device according to claim 1, wherein the plurality of short pins (43) have elasticity.
